# EUROPEAN PATENT SPECIFICATION

(11) **EP 2 647 339 B1**
(45) Date of publication and mention of the grant of the patent: **22.04.2020**
(21) Application number: 13174810.5
(22) Date of filing: 18.08.2009
(51) Int. Cl.: A61B 17/32, A61B 17/16, A61B 90/98, A61B 17/00

(54) **Arthroscopic shaver**
Arthroskopisches Resektionsinstrument
Appareil de résection arthroscipique

(30) Priority: 19.08.2008 GB 0815136
(43) Date of publication of application: 09.10.2013
(62) Divisional of application: 09252013.9
(73) Proprietor: De Soutter Medical Limited, Buckinghamshire HP22 5WF (GB)
(72) Inventor: De Soutter, George Charles, Aylesbury Bucks HP22 5WF (GB); Croft, Jonathan, Aylesbury Bucks HP22 5WF (GB)
(74) Representative: Granleese, Rhian Jane

(56) References cited:
- EP-A1- 0 947 167
- EP-A1- 1 356 776
- WO-A1-97/12554
- DE-U1- 29 708 720

## Description

The invention relates to the field of medical devices and specifically to powered surgical instruments.

Powered surgical instruments, for example, mechanical cutters are typically motor driven. Such instruments are often manufactured as detachable from the motor. The part containing the motor is for example a handpiece that allows a surgeon or operator to control the medical instrument. A handpiece may be usable with a number of interchangeable instruments. For example, a cutting tool may have a number of interchangeable blades and a drill may be useable with a number of drill bits. Different devices and blades may require different driving conditions. To facilitate proper functioning of the instrument it is advantageous to incorporate a facility to determine a property of a surgical instrument in a handpiece for driving the surgical instrument.

An example of an area of surgery that uses powered surgical instruments is arthroscopic surgery. Arthroscopic surgery is a minimally invasive technique for the surgery of joints. During arthroscopic surgery an arthroscopic shaver blade is inserted into a joint of a patient and used to remove or reshape tissue or bone. Arthroscopic shavers generally consist of two tubular components: an outer portion and an inner portion that rotates within the outer portion. The shaver is coupled at a proximal end to a handpiece that drives the inner portion and causes it to rotate within the outer portion. The outer portion has a cutting window in the distal end that exposes a cutting part of the inner portion. In operation, a surgeon manipulates the handpiece to bring the cutting window into proximity of region of tissue to be operated on and the rotating cutting part exposed to the tissue removes part of the tissue.

The wide range of possible arthroscopic surgery procedures means a variety of shaver blades are used. Shaver blades can vary by the style of cutting window; different cutting windows are used to remove different tissue types. The shape of the blade can also vary; blades with a straight shaft are commonly used while curved blades are used to reach otherwise inaccessible area. The cutting part of the end portion can also vary; shavers are used to remove soft tissue and burrs are used to reshape bone.

The different varieties of shaver blades have different working conditions such as range of operating speeds, required torque output, and maximum operating speed. Thus different shaver blades may require different driving conditions from the motor.

To achieve the desired cutting effect a power supply to the motor may require manual adjustment. This is time consuming for staff setting up an operating procedure, is also subject to human error and may make conditions difficult to standardise between operations. Further, a change of blade during a surgery requires the power supply to be adjusted during the surgery leading to an extension of the time required for the surgery.

The handpiece may include a shaver blade recognition means to determine the type of shaver blade and relay this information to a power supply to determine the output characteristics of the motor and control the driving conditions of the blade.

US patent 5,632,759 discloses a disposable cutting blade assembly for use with a handpiece having a motor for rotatably driving the cutting blade assembly. The cutting blade assembly includes a plastic hub mounted on a proximal end of an outer member. In a disclosed embodiment magnets are disposed in the plastic hub and reed switches are disposed in the handpiece to effect speed range control.

DE29708720 describes a surgical motor.
EP1356776 describes an identification system for moving tool bits in medical devices, for example for application in arthroscopies.
WO97/12554 describes a surgical instrument with embedded coding elements. EP0947167 describes methods and apparatus to recognise surgical apparatus

The inclusion of magnets in the proximal end of a shaver blade allows the type of shaver blade to be recognised by the handpiece, and thus the power to the handpiece to be automatically controlled. The presence of magnets in the shaver has a number of undesirable effects.

The magnets increase the diameter of the shaver. Cylindrical magnets having a diameter of approximately 2.5mm and a length of 1.5 to 2mm are often used. Because such magnets are produced by sintering, this is the minimum size in which they can be produced. Further, the magnets must be large enough to produce a magnetic field strong enough to be detected in the handpiece. The magnets are located around the hub portion and must be located away from the central axis of the shaver blade that is occupied by the inner portion of the shaver blade. This has the effect that the exterior radius of the hub portion of the shaver blades must be at least 1.5 mm greater than the internal radius of the opening. Generally, the exterior of the hub portion covers the magnets to secure the magnets; this further increases the necessary diameter of the shaver blade. This increases the necessary diameter of the part of the handpiece that accommodates the shaver. This limits the design of the handpiece and increases the necessary overall diameter of the handpiece.

Further, the manufacture of the shaver blades is made more complex by the inclusion of magnets. The magnets may be over moulded in an injection moulding process to manufacture the coupling of the shaver blade. However, this process may be unreliable leading to a misalignment of the magnets relative to the sensory components in the handpiece and shaver blades may be misrecognised. An alternative to over moulding is manually inserting the magnets into the coupling part of the shaver blade. This leaves the end product open to human error, which may lead to misrecognition of shaver blades by the handpiece.

It is therefore an object of the present invention to provide for the recognition of a surgical instrument by a handpiece without at least some of the problems described above resulting from the inclusion of magnets on the surgical instrument.

The invention is disclosed by independent claim 1, with preferred embodiments disclosed in the dependent claims.

According to an aspect of the present invention there is provided an arthroscopic shaver blade as surgical instrument. The surgical instrument is adapted to be mounted on a driving means. The surgical instrument comprises an indicator means for allowing the driving means to determine a property of the surgical instrument. The indicator means comprises a soft magnetic material configured to affect a magnetic field detected in the driving means.

The surgical instrument may be, for example, a component for a rotational cutting instrument.

The indicator means comprises a material that can conduct or otherwise manipulate an external magnetic field such that the shape and/or type of the material allows a property of the instrument to be identified. For example, the property may be the type of surgical instrument. The shape or configuration of the soft magnetic material would then allow the driving means to identify the type of surgical instrument. Further, different soft magnetic materials having different magnetic conductivities may be used as a further means of identification.

Having a soft magnetic material disposed on a surgical instrument means that the surgical instrument can be identified by applying a magnetic field to the instrument. This means that magnets do not have to be included on the surgical instrument. Instead, the magnets are included in a handpiece for use with the surgical instrument. This has a number of advantages: the coupling portion of the surgical instrument can be smaller as magnets do not have to be accommodated. The soft magnetic material diverts the magnetic field applied to it. The soft magnetic material does not have to be as thick as the magnets to allow recognition of the surgical instrument. This allows the instruments having a smaller diameter to be realised. This allows a smaller, more ergonomic handpiece and makes instrument recognition possible in a wider range of arthroscopic surgeries. Further, as the surgical instruments are often disposable and the handpiece is not, the inclusion of magnets in the handpiece rather than the surgical instrument has a potential cost saving.

According to an embodiment of the present invention, the soft magnetic material is non-structural.

According to the present invention, the soft magnetic material is arranged substantially in a plane. The plane being substantially perpendicular to a longitudinal axis of the surgical instrument.

According to the present invention, the soft magnetic material is disposed around longitudinal axis of the surgical instrument.

The extent as well as the presence of the soft magnetic material may be used in the identification of the surgical instrument, for example, a half rotation of the soft magnetic material can be used for the identification of the surgical instrument

According to the present invention, the soft magnetic material is a ring section located around the longitudinal axis. The ring section may have an opening subtending an angle of at least 40 degrees at the longitudinal axis.

According to an embodiment of the present invention, the soft magnetic material is configured to fit inside the driving means.

According to an embodiment of the present invention, the soft magnetic material forms part of the surface of the surgical instrument. This enables the distance between the soft magnetic material and the magnets and sensors in the handpiece to be minimised. Thus the effect of the diversion of the magnetic field is maximised. The detection of the diversion and therefore the determination of the property of the surgical instrument can thus be made more accurately.

According to an embodiment of the present invention, the surgical instrument comprises orientating means. The orientating means may fix the angular orientation of the surgical instrument relative to the handpiece. Fixing the angular orientation of the surgical instrument makes it possible to fix the position of the soft magnetic material relative to magnets and sensors located in the handpiece.

According to an embodiment of the present invention there is provided a plurality of interchangeable surgical instruments. Each instrument of the plurality of surgical instruments is configured to be interchangeably mounted on a common driving means. Each instrument has a difference in a property and each indicator means is configured to differentiate between instruments to indicate said difference in said property.

In the following, embodiments of the present invention will described as non-limiting examples with reference to the figures in which:
FIG.1 shows a side view of a surgical instrument and a handpiece;
FIG.2 shows a cross section of a surgical instrument and a handpiece;
FIG.3 shows a cross section of a coupling means of a handpiece having a surgical instrument inserted into it;
FIG.4 shows a magnetic field resulting from magnets in a handpiece;
FIG.5 shows a magnetic field resulting from magnets in a handpiece and modified by a soft magnetic material;
FIG.6 shows a magnetic field resulting from magnets in a handpiece and modified by a soft magnetic material;
FIG.7 shows a graph of Hall sensor voltage measured in a handpiece against distance between a magnet and a soft magnetic material on a shaver blade coupled to the handpiece;
FIG.8 shows a graph comparing different soft magnetic materials;
FIG.9 shows a graph of Hall voltage and the size of a split in a ring of a soft magnetic material;
FIG.10 shows a graph of Hall voltage and the angle of a split in a ring of a soft magnetic material;
FIG.11 shows a side view of an arthroscopic shaver attached to a handpiece;
FIG.12 shows a sectioned side view of an arthroscopic shaver attached to a handpiece;
FIG.13 shows an isometric view of the proximal end of shaver blade;
FIG.14 shows an expanded isometric view of the proximal end of a shaver blade;
FIG.15 shows a sectioned side view of the proximal end of a shaver blade;
FIG.16 shows an expanded isometric view of the proximal end of a shaver blade;
FIG.17 shows the geometry of a section of soft magnetic material;
FIG.18 shows a sectioned side view of the front of a handpiece connected to a shaver;
FIG.19 shows a sectioned top view of the front of a handpiece connected to a shaver;
FIG.20 shows a vertical section of a handpiece; and
FIG.21 shows a vertical section of a handpiece.

While the description of determination of a property of a surgical instrument given below relates to shaver blades, it will be apparent to one of skill in the art that the use of a soft magnetic material in the determination of a property of a surgical instruments is not limited to shaver blades.

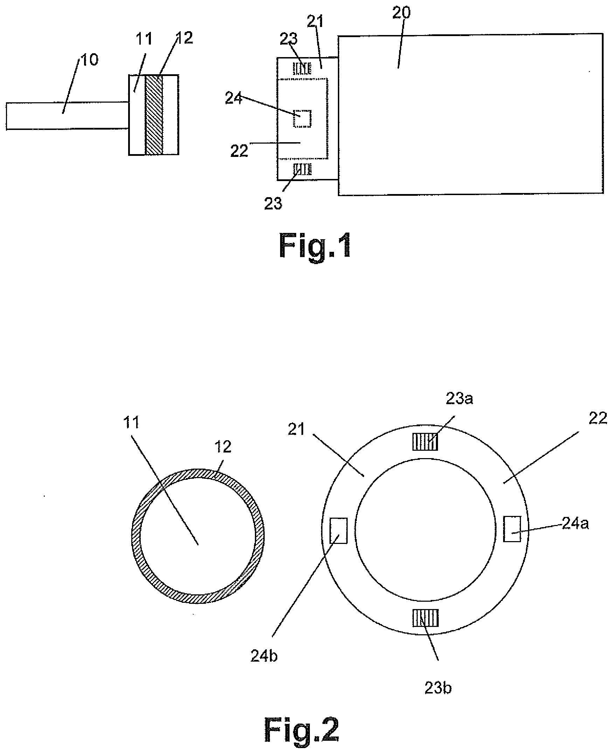

FIG.1 shows a surgical instrument 10 and a handpiece 20. In operation, a motor in the handpiece drives the surgical instrument 10. The surgical instrument 10 has a coupling portion 11 that attaches to the handpiece 20. A ring of soft magnetic material is located on the coupling portion of the surgical instrument. When the surgical instrument is inserted into the handpiece 20, the soft magnetic material diverts a magnetic field applied to it by magnetic field sources in the handpiece 20.

The handpiece 20 has a coupling means 21 for coupling to the surgical instrument 10. The coupling means 21 has an opening 22 to receive the surgical instrument 10. When the surgical instrument is coupled to the handpiece 20, the handpiece 20 can drive the surgical instrument 10. The coupling portion 11 of the surgical instrument 10 may not move relative to the handpiece 20. For example, the surgical instrument 10 may comprise more than one part and the handpiece 20 may cause part of the surgical instrument 10 to move relative to another part, this is the case in an arthroscopic shaver blade as described in more detail below.

The coupling means 21 of the handpiece 20 has an opening 22 to receive the surgical instrument 10. The opening 22 is substantially circular in cross section. Magnetic field sources 23 are located around the opening to apply a magnetic field to a surgical instrument inserted into the opening. Magnetic field sensors 24 are also located around the opening to measure the magnetic field at these points.

FIG.2 shows a cross section of the coupling portion 11 of the surgical instrument 10 and the coupling means 21 of the handpiece 20. The coupling portion 11 of the surgical instrument 10 has a ring of soft magnetic material 12 located around it. The shape and extent of the soft magnetic material indicates a property of the surgical instrument 10. The soft magnetic material 12 is non-structural.

The coupling means 21 of the handpiece 20 has magnetic field sources 23a, 23b and magnetic field sensors 24a, 24b located around the opening 22 that receives the surgical instrument 10. The coupling means 21 of the handpiece 20 has two magnetic field sources 23a, 23b on opposite sides of the opening 22. The coupling means 21 of the handpiece has two magnetic field sensors 24a, 24b on opposite sides of the opening 22.

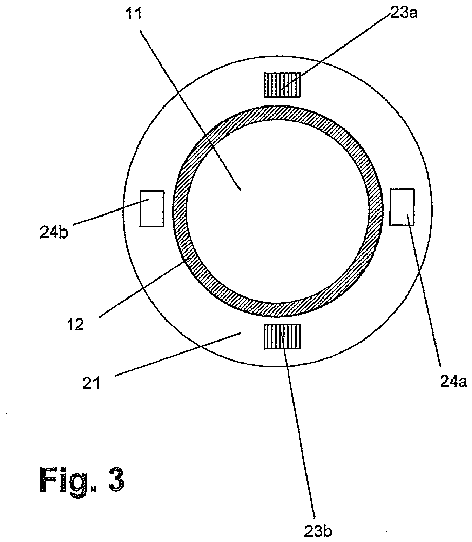

FIG.3 shows a cross section of the coupling means 21 of the handpiece 20 with the coupling portion 11 of the surgical instrument 10 inserted in the opening 22. The ring of soft magnetic material 12 runs through a location below the magnetic field source 23a located at the top of the opening 22, the magnetic field source 23b located at the bottom of the opening 22. Similarly, the ring of soft magnetic material 12 runs through a location above the magnetic field sensor 24a located at the right hand side of the opening 22 and the magnetic field sensor 24b located at the left hand side of the opening 22.

The soft magnetic material 12 indicates a property of the surgical instrument 10 from its shape, This property is determined by the handpiece 20 as follows. The magnetic field sources 23a, 23b apply a magnetic field to the coupling portion 11 of the surgical instrument 10. The soft magnetic material 12 diverts this magnetic field past the magnetic field sensors 24a, 24b. Thus, the soft magnetic material 12 modifies the magnetic field detected by the magnetic field sensors 24a, 24b. The magnetic field strengths measured by the magnetic field sensors 24a and 24b are determined by the shape and/or type of the soft magnetic material. From the shape of the soft magnetic material, the property of the surgical instrument is determined.

The property indicated by the soft magnetic material 12 is for example the type of surgical instrument. The property may be the maximum driving speed or a range of operating speeds for the surgical instrument 10. By measuring the magnetic field using the magnetic field sensors 24a, 24b, this property is determined by handpiece 20.

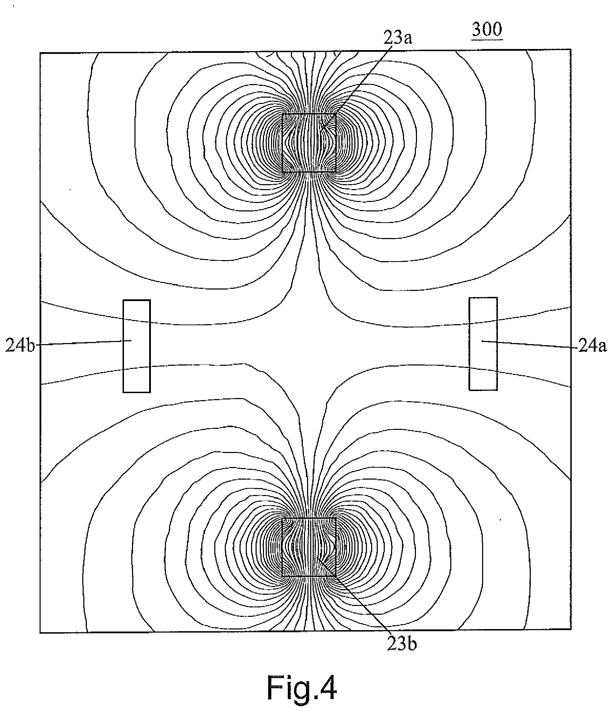

FIG.4 shows the magnetic field 300 resulting from the magnetic field sources 23a, 23b located in the coupling means 21 of the handpiece 20. FIG.4 shows to a cross section of a handpiece without a surgical instrument inserted, or having a surgical instrument inserted without a soft magnetic material disposed around it. A magnetic field source 23a is at the top of the opening in the coupling portion in the handpiece directly above a second magnetic field source 23b at the bottom of the opening. The magnetic field sources are positioned so that opposing magnetic poles face each other. For example, the North pole of magnetic field source 23a at the top of the opening faces downwards towards the magnetic field source 23b located at the bottom of the opening and the North pole of the magnetic field source 23b located at the bottom of the figure faces upwards. The magnetic field sensors 24a, 24b occupy positions halfway up the opening at the left and right hand sides.

The magnetic field 300 is shown as a number of magnetic field lines. The magnetic field lines run from one pole of either magnet to a complementary pole. It is noted that the magnetic field lines do not run from one magnet to the other. This is because the magnets are positioned with opposing poles facing each other. The strength of the magnetic field 300 is shown by the closeness of the magnetic field lines. Where the magnetic field lines are close together, the magnetic field is stronger than in regions where the magnet field lines are further apart.

In FIG.4, the magnetic field is strong in the regions close to the magnetic field sources, but relatively weak elsewhere, including the region around the magnetic field sensors. Both the right hand magnetic field sensor 24a and the left hand magnetic field sensor 24b would detect relatively low magnetic field strengths.

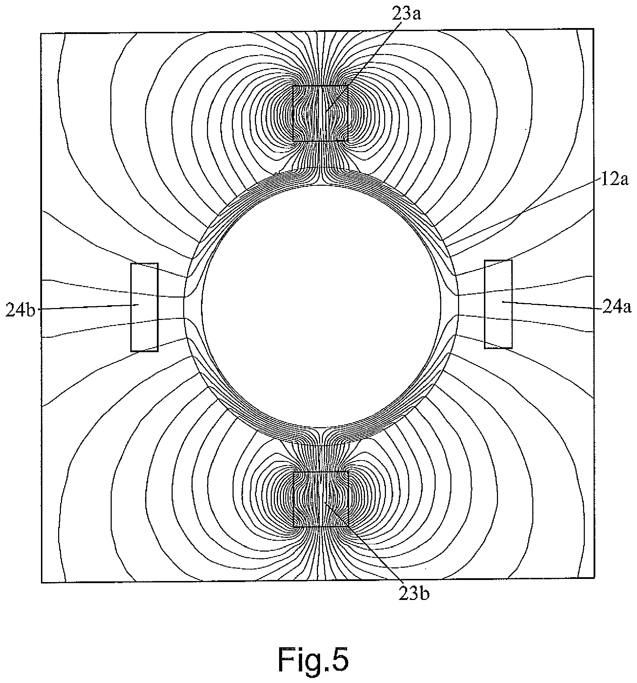

FIG.5 shows the magnetic field 302 resulting from the magnetic field sources 23a, 23b located in the coupling means 21 of the handpiece 20 and channelled by a ring 12a of soft magnetic material. The ring 12a of soft magnetic material completes a full circle thus forming a ring shape in the opening in the coupling means for the handpiece. Above and below the ring 12a of soft magnetic material are the magnetic field sources 23a, 23b. To the left and right of the ring 12a of soft magnetic material are the magnetic field sensors 24a, 24b.

The presence of the ring 12a of soft magnetic material modifies the magnetic field 302. Because the ring 12a of soft magnetic material conducts the magnet field applied to it, the magnetic field within the ring 12a of soft magnetic material is strong; the magnetic field lines within the ring 12a of soft magnetic material are close together. In the area inside the ring 12a of soft magnetic material the magnetic field is close to zero. Considering the left hand side of the ring 12a of soft magnetic material, the magnetic field from the magnetic field source 23a at the top of the opening meets the magnetic field from the magnetic field source 23b at the bottom of the opening at locations in the ring 12a of soft magnetic material adjacent to the magnetic field sensors 24a 24b.

Comparison of FIG.4 and FIG.5 shows that due to the presence the ring 12a of soft magnetic material, the magnetic field 302 is stronger at the locations of the magnetic field sensors 24a, 24b. Thus, a measurement of a relatively high magnetic field at both of the magnetic field sensors indicates that a ring 12a of soft magnetic material is present.

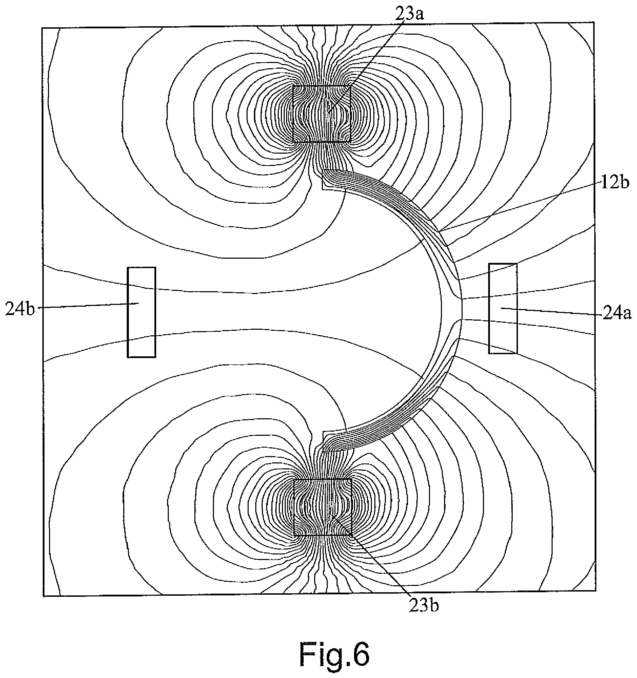

FIG. 6 shows the magnetic field 304 due to the magnetic field sources 23a, 23b and a half ring 12b of soft magnetic material. The layout of the magnetic field sources 23a, 23b and the magnetic field sensors 24a, 24b is as described in reference to FIG.4 and FIG.5. The soft magnetic material forms a semi circular half ring 12b section running from a location below the top magnetic field source 23a through a location adjacent to the magnetic field sensor 24a on the right hand side of the opening in the coupling means of the handpiece to a location above the magnetic field source 23b at the bottom of the opening.

The magnetic field 304 is stronger in the region surrounding the magnetic field sensor 24a on the right hand side of the opening than in the region surrounding the magnetic field sensor 24b on the left hand side of the opening. This is because the half ring 12b of soft magnetic material acts to conduct the magnetic flux from the magnetic field sources to a location adjacent the right hand magnetic field sensor 24a. As there is no soft magnetic material located in the vicinity of the left hand magnetic field sensor 24b the magnetic field strength there is lower. In the arrangement shown in FIG. 6 the right hand side magnetic field sensor would detect a relatively high magnetic field strength and the magnetic field sensor 24b located on the left hand side would detect a relatively low magnetic field strength.

The soft magnetic material indicates a property of the surgical instrument. The values of the outputs from the magnetic field sensors are used to determine the property of the surgical instrument. With no soft magnetic material located on the surgical instrument as shown in FIG. 4, both of the sensors would detect a relatively low magnetic field strength. Locating a ring of soft magnetic material around the coupling portion 11 of the surgical instrument 10 would cause both of the magnetic field sensors to detect a relatively high magnetic field. A half ring causes one of the magnetic field sensors to detect a relatively high magnetic field and the other to detect a relatively low magnetic field.

The half ring 12b of soft magnetic material, as shown in FIG.6 causes one magnetic field sensor to detect a high magnetic field and the other to detect a low magnetic field. Such an arrangement of soft magnetic material may require an orientating means on the coupling portion of the surgical instrument and in the coupling means of the handpiece to fix the rotational orientation of the surgical instrument. There are two possible orientations of the surgical instrument that would result in a high magnetic field at one of the magnetic field sensor and a low magnetic field at the other sensor. These possible orientations are 180 degrees apart. The orientating means may fix the surgical instrument in one of these possible orientations. This may be beneficial to a user of the medical device as the two orientations may provide increased flexibility when used for arthroscopic procedures.

Thus, the shape of the soft magnetic material located on the coupling portion of the surgical instrument is used to indicate a property of the surgical instrument. In the example above, three different possible arrangements are discussed; a property may be indicated as one of three possible values. A further set of magnetic field sources and magnetic field sensors could be included in the handpiece and a further ring or ring sections could be included on the surgical instrument to increase the number of possible indications. Further, different arrangements of the magnetic field sources and magnetic field sensors may be used. Further, a range of materials could be used, each having different magnetic conduction properties.

Different shapes of magnetic material could be used. To determine a property of the surgical instrument, the handpiece detects a modification in the magnetic field due to the soft magnetic material. Therefore any shape of soft magnetic material that will modify the magnetic field detected by the sensors can be used to indicate a property of the surgical instrument.

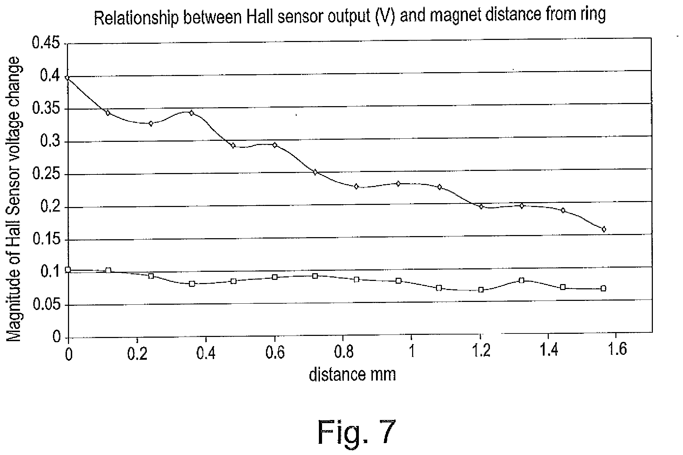

FIG.7 shows a graph of Hall sensor voltage change against distance for a handpiece as described above. The magnetic field sensor used is a radiometric Hall effect sensor having a sensitivity of 50V/T. The magnetic field sources used were cylindrical Samarium Cobalt Series 2:17 magnets having a diameter of 2mm and a length of 4.4mm. The diamond symbols indicate the voltage change against the distance from the magnet for a shaver blade having a hardened 440 stainless steel ring as a soft magnetic material. The square symbols indicate the voltage change for a surgical instrument without a soft magnetic material around the coupling portion, instead having a Acetel ring. The results show that even at distances of 1.5mm from the magnets, the presence of the soft magnetic material ring is detectable. This shows that the use of a soft magnetic material for the identification of surgical instruments is possible within the dimensions of existing surgical handpieces devices.

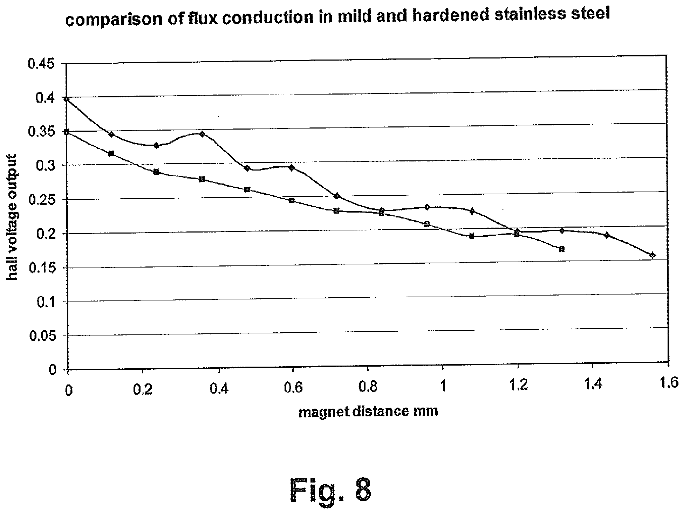

FIG.8 is a graph showing a comparison of soft magnetic materials. The points shown as diamonds indicate the Hall voltage against distance from the magnets for a ring of hardened stainless steel. The points shown as squares indicate Hall voltage against magnet distance using a mild steel (EN1A) ring. It is noted that at distances of 1.5mm from the magnets the presence of a ring of hardened stainless steel or mild steel would be detectable over the Acetel ring, results for which are shown in FIG.7.

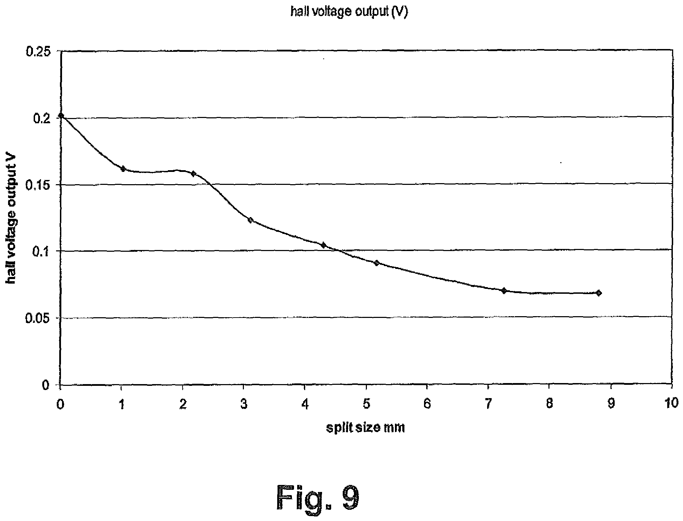

FIG.9 shows a graph of split size and Hall voltage for a mild steel (EN1A) ring. The split size on the x-axis refers to the size of a split in the ring adjacent to one of the Hall sensors as shown in FIG. 5. The results were produced having a separation of 1mm between the magnets and the ring and a separation of 1.15mm between the Hall sensors and the ring. The measurements shown are the Hall voltages from the Hall sensor adjacent to the split, shown as 24b in FIG.6. The graph shows that the Hall voltage is approximately half the value with no split (i.e. a full ring) when the split size is 4mm. Thus a split of approximately 4mm in the ring results in a voltage difference that can be reliably detected and therefore split are suitable for indicating a property of the surgical instrument.

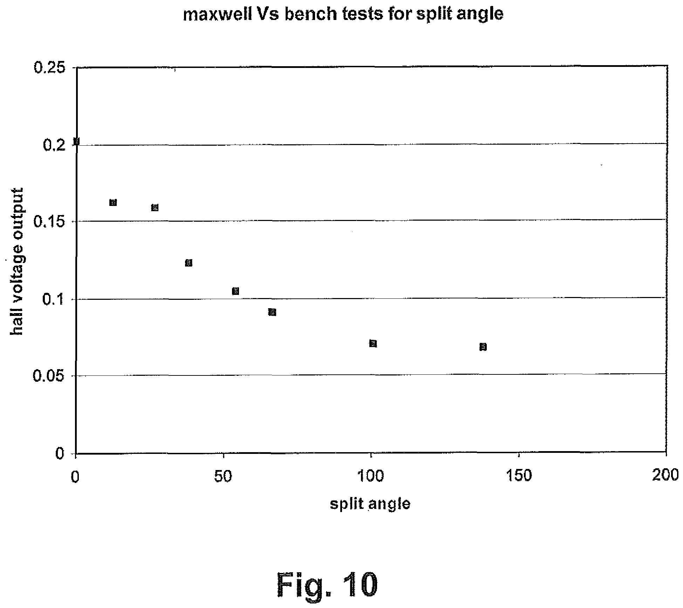

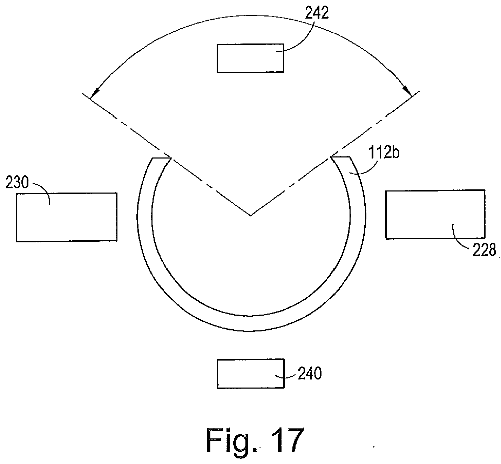

FIG.10 shows a graph of Hall voltage against split angle for the same apparatus as the graph in FIG.9. The graph shows that the Hall voltage output has a value of approximately 0.2V with no split. The Hall voltage decreases to 0.1V for a split angle of approximately 60 degrees. For split angles of greater than 60 degrees, the Hall voltage begins to level off and levels off to a value of approximately 0.07V for split angles of greater than 110 degrees. It is noted in reference to FIG. 7 that the Hall voltage in the presence of a soft magnetic ring for a magnet distance of 1mm is approximately 0.22V and the Hall voltage without a soft magnetic ring is approximately 0.07V. The graph in FIG. 10 shows that split angles of greater than 40 degrees can be detected. The implications of these results are further discussed below in relation to FIG.17.

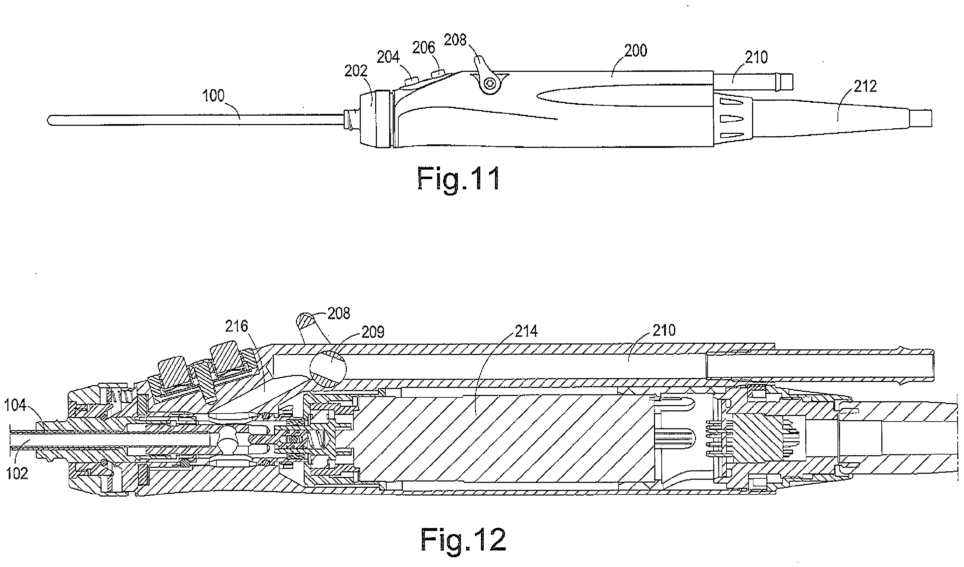

FIG.11 shows a side view of an arthroscopic shaver 100 attached to a handpiece 200. The handpiece 200 is substantially cylindrical in construction with a blade release sleeve located 202 at one end. The blade release sleeve 202 is circular and allows the shaver 100 to be decoupled from the handpiece 200. The shaver 100 is a surgical instrument that can be removed by pushing the blade release sleeve 202 towards the body of the handpiece 200. The shaver 100 is attached to this end of handpiece 200. Shaver 100 is tubular in construction and extends from distally from a proximal end attached to handpiece 200.

Located on the top of the handpiece 200, close to blade release sleeve are two control buttons 204, 206. The control buttons allow control of a motor within the handpiece 200. Also located on the top of handpiece 200 is a suction control lever 208. The suction control lever 208 is attached to the handpiece 200 by a pivot and can be moved through an angle of approximately 90 degrees. Extending from the rear end of handpiece 200 is a suction tube 210 and a control cable 212.

FIG.12 shows a sectioned side view of the shaver 100 attached to the handpiece 200. The shaver 100 has an inner tubular section 102 located inside an outer tubular section 104.. Within the handpiece 200 is a motor 214. The motor 214 engages with the inner tubular section 102 of the shaver 100. The suction tube 210 extends internally from the rear end of the handpiece 200 along the top of the handpiece 200 to a diagonal suction tube section 216 that connects the suction tube 210 to the inner tubular portion 102 of the shaver 100. Valve 209 allows the suction to be regulated by positioning the suction control lever 208.

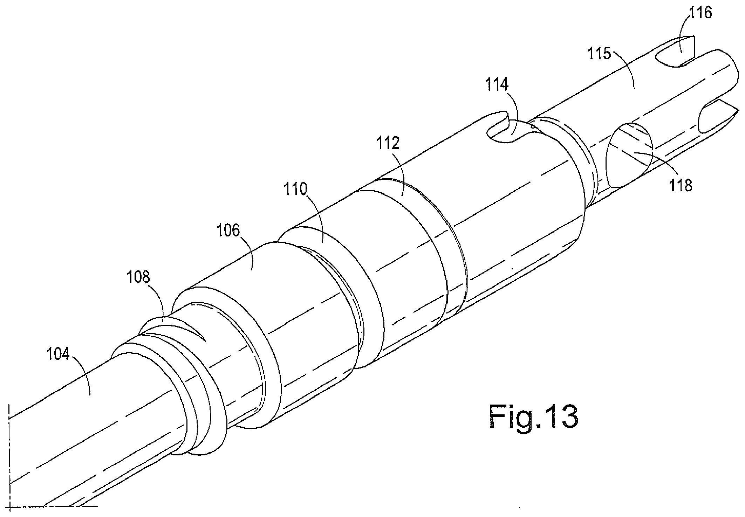

FIG.13 shows an isometric view of the proximal end of a shaver blade 100. The outer tubular section 104 has a coupling portion 106. The coupling portion 106 and the outer tubular section 104 have a common axis. The coupling portion 106 is substantially cylindrical and has a diameter greater than that of the remainder of the outer tubular portion 104. Running around a circumference of the coupling portion 106, at the end attached to length of the tubular section 104 is a thread 108. A groove 110 is located around a circumference of the coupling portion 106. The groove 110 allows the shaver 100 to be secured to the handpiece 200 in the correct location. Located around at least a part of a circumference of the coupling portion 106 is a soft magnetic material 112. The soft magnetic material 112 is a material that conducts a magnetic field applied to the coupling portion 106. At the end of the coupling portion 106 is an orientation slot 114. The orientation slot 114 is a groove running along the surface of the coupling portion 106 parallel to the axis of the coupling portion 114 and the outer tubular portion 104.

The soft magnetic material 112 allows the type of surgical instrument to be identified by the handpiece 200. The soft magnetic material 112 diverts a magnetic field applied to the coupling portion 106 by magnets within the handpiece. Magnetic field sensors within the handpiece measure the strength of the magnetic field at locations adjacent to locations on the coupling portion 106, and from this magnetic field strength the handpiece determines the extent or location of the soft magnetic material. Therefore different configurations of soft magnetic material on the surgical instrument can be distinguished. This is used to identify the type of shaver blade or surgical instrument inserted in the handpiece.

Attached to the proximal end of the inner tubular portion 102 is a coupling portion 115 of the tubular inner portion 102 that has a circular hole 118 running across its tubular section. This allows debris from the surgical site to flow through inner tubular portion 102 and connect to the irrigation means of the handpiece 200. The end of the coupling portion 115 has four slits 116 which run parallel to the axis of the inner tubular portion 102 and four teeth on the end of the tubular portion 102. This allows the tubular portion 102 to be driven by a motor 214 located within the handpiece 200.

The shaver and the handpiece are used to perform surgery as follows: The shaver is inserted into a patient's joint cavity. The joint space is caused to distend by introducing an irrigation fluid. This allows a surgeon to view the shaver inside the patient using a visualisation instrument such as a camera attached to a viewing screen. Such visualisation instruments are known as arthroscopes. The shaver blade is placed over an area of the patient's joint to be modified and the inner tubular portion is caused to rotate within the outer tubular portion, which removes tissue or reshapes bone. The handpiece applies a magnetic field to the coupling portion of the shaver blade and the soft magnetic material diverts this magnetic field. Based on this diversion of the magnetic field, a property of the shaver blade is determined. The property is a range of speeds for the shaver blade or a maximum speed for the shaver blade. The speed of operation of the shaver blade is controlled using the property.

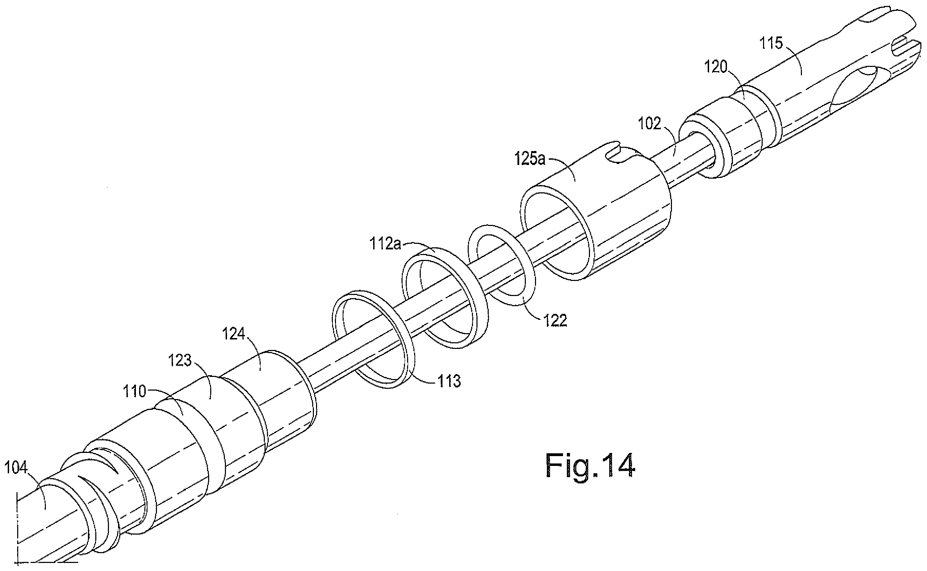

FIG. 14 shows an expanded view of the construction of a coupling portion of a shaver blade. The inner tubular portion 102 fits inside the outer tubular portion 104. The inner tubular portion is attached to a coupling portion 115, which has a groove 120 around its circumference and is as described above with reference to FIG. 13. The coupling potion attached to the outer tubular portion 104 is constructed from two parts: a front portion 123 and a rear portion 125a. The front portion 123 is cylindrical and attached to the outer tubular portion 104 with which it has a common longitudinal axis. There is a groove 110 around the circumference of the front portion 123. The end of the front portion distal to the outer tubular portion 104 has a recessed portion 124, which has an exterior radius less than that of the rest of the front portion 123. Identification ring 113 fits over the recessed portion 124 and is made from coloured plastic to allow identification of the shaver blade. Soft magnetic material ring 112a also fits over the recessed portion of the front portion 123. O-ring 122 is located around inner tubular portion 102 and has a radius approximately equal to the recessed portion 124 of the front portion 123. Rear portion 125a fits over the recessed portion 124 of the front portion 123.

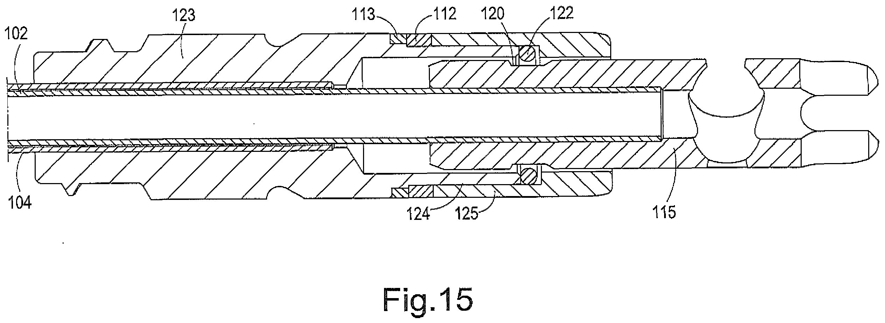

FIG. 15 shows a cross section of the shaver blade described above with reference to FIG. 14. The inner tubular portion 102 fits the inside outer tubular section 104. The inner tubular section 102 extends beyond the end of the outer tubular section 104. The coupling portion 115 of the inner section 102 is attached over the end section of the inner tubular section 102 that extends along the longitudinal axis of the two tubular sections from the outer tubular section 104. The coupling portion 115 of the inner tubular section 102 has a groove 120 around its circumference that engages with o-ring 122 to prevent the inner tubular section 102 from falling out of the outer tubular section 104 when the shaver blade is handled.

The coupling portion of the outer tubular section 104 has a front portion 123 which cylindrical and attached over outer tubular section 104. The coupling portion of the outer tubular section forms a cylindrical cavity that receives the coupling portion of the inner tubular section. This cavity is formed from the inside of the front portion 123 and part of the inside of the rear portion 125. The identification ring 113 and the soft magnetic material 112 fit over the recessed section 124 of front portion 123. The identification ring 113 and the soft magnetic material 112 have an exterior radius equal to those of both the exterior surfaces of the front and rear coupling portions. The rear portion 125 has a section sized internally to receive the recessed section 125 of the front portion 123. This continues beyond the end of the recessed section 125 to form a groove that accommodates the o-ring 122.

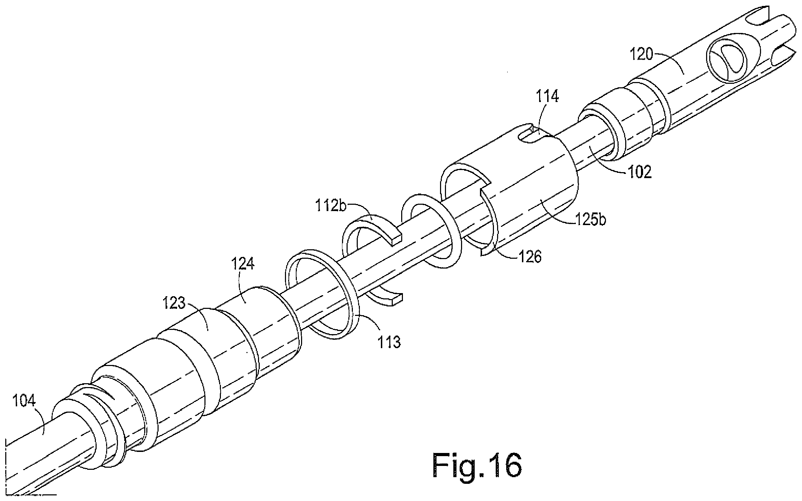

FIG. 16 shows an expanded view of a coupling portion of a shaver blade having a split ring. The inner tubular section 102, the coupling portion 120 of the inner tubular section, the outer tubular section 104 and the front portion 123 of the coupling portion of the outer tubular section are as described with reference to FIG. 14. The indicator ring 113 and the soft magnetic material ring section 112b fit over the recessed section of the front section 123 of the coupling potion of the outer tubular section 104. The split ring 112b of soft magnetic material is centred on the longitudinal axis of the inner and outer tubular sections and has an open section subtending an angle of 110 degrees on the longitudinal axis. The rear portion 125b of the coupling portion has a tab 126 that protrudes in the direction of the longitudinal axis of the shaver in the distal direction. The tab 126 is shaped to fill the open section of the split ring 112b of soft magnetic material. The tab 126 acts as a spacer and ensures that the split ring 112b of soft magnetic material maintains its rotational position.

The split ring 112b and the rear portion 125b of the coupling portion are aligned with the cutting window of the shaver blade. The orientation of the coupling portion is fixed relative to the handpiece by the orientation slot 114 on the rear portion of the coupling portion.

FIG. 17 shows the geometry of the split ring section 112b relative to magnets and sensors in the handpiece. The handpiece has two magnets 228, 230 located at on each side of the opening that receives the shaver blade. Also at opposing sides of the opening there are two Hall sensors 240, 242. The split ring 112b of soft magnetic material is located on the coupling portion of a shaver blade that is coupled to the handpiece. When the shaver blade is coupled to the handpiece, the soft magnetic material is located in the same plane as the magnets and the Hall sensors as is shown in FIG. 17.

The split is located below one of the Hall sensors 242. The split has an angle of 110 degrees. In reference to FIG. 10, it is noted that the Hall voltage is roughly constant for split angles of up to 110 degrees at the sensor adjacent to the split and for angles less than 110 degrees, the Hall voltage increases as the split angle decreases. A split of 110 degrees is considered optimum. Decreasing the split size from 110 degrees would make the Hall voltage on the sensor adjacent to the split increase and therefore make the process of differentiation between a full ring and a split ring less accurate. Making the split larger would not greatly affect the Hall voltage, but would have the detrimental effect of reducing the strength of the fastening between the split ring of soft magnetic material and the coupling portion of the shaver blade.

The optimum split angle of 110 degrees relates to the particular dimensions and properties of the Hall sensor. FIG. 10 shows that split angles of 40 to 180 degrees, and could be used to determine the presence of a split and indicate a property of the shaver blade. Preferably, the split angle is between 60 and 160 degrees.

The thickness of the ring and split ring of soft magnetic material is preferably less than 2mm and more preferably less than 1mm.

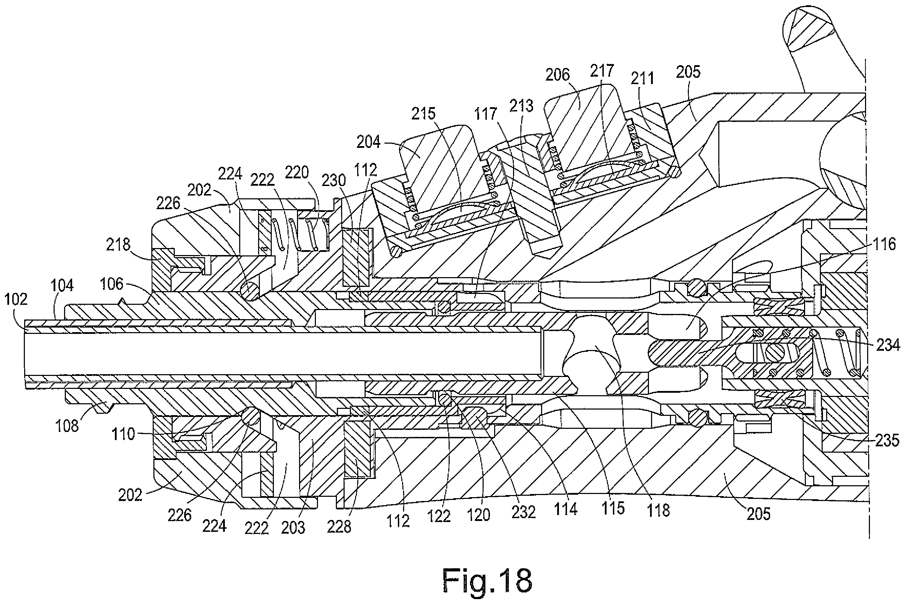

FIG. 18 shows a section of the side of the front portion of the handpiece with a shaver attached. The inner tubular portion 102 of the shaver is located within the outer tubular portion 104. The coupling portion 106 of the outer tubular portion 104 is substantially cylindrical and is located around the outer tubular portion 104. The coupling portion 106 extends beyond the end of the outer tubular portion into the handpiece. On the inside face of the coupling portion 106, an O-ring 122 is located around the inside of the coupling portion at a location beyond the end of the outer tubular portion 104. The O-ring 122 engages with a groove 120 that is located around the outside of the coupling portion 115 of the inner tubular portion 102 to stop the inner tubular portion from falling out when the shaver blade is handled. The coupling portion 115 is attached over the proximal end of the inner tubular portion 102. The coupling portion 115 has a tubular cavity with connects the inner tubular portion 102 with a circular hole 118 in the side of coupling portion 115.

The shaver housing 203 of the handpiece defines a cylindrical cavity for receiving the coupling portion 106 of the outer tubular portion 104 and the coupling portion 115 of the inner tubular portion of a shaver blade. The outer body 205 of the handpiece fits around the shaver housing 203. The shaver housing 203 extends beyond outer body 205 and is covered by the blade release sleeve 202. A thread 108 runs around the circumference of the coupling portion 106 of the outer tubular portion 104 in front of the blade release sleeve 202.

The shaver housing 203 is attached to an annular lip 218 which projects outwards and limits the movement of the blade release sleeve 202 away from the body of the handpiece 200.

A spring 220 is located within the blade release sleeve 202 and urges the blade release sleeve 202 away from the body of the handpiece. The shaver housing 203 of the handpiece 200 has two channels 222 that run away from the cavity at an angle of approximately 60 degrees from the radial direction of the cylinder defined by the handpiece. A washer 224 is attached to the blade release sleeve 202 such that it limits the movement of the two pins 226 within the channels 222. The Two pins 226 engage with a groove 110 in the coupling portion 106 of the outer tubular portion 104 of the shaver blade 100.

When the blade release sleeve 202 is pushed towards the handpiece, the washer 224 moves with the blade release sleeve 202 towards the body of handpiece. This allows the pins 226 to move in the channels 222. This movement of the pins 226 has the result that they no longer engage with the groove 110 in the coupling portion of the outer portion of the shaver. Therefore, the shaver can be removed from the handpiece.

Two magnets 228, 230 are located between the shaver housing 203 and the outer body 205 of the handpiece. The magnets 228, 230 are located in opposing positions with respect to the cavity formed by the shaver housing 203. The magnets 228, 230 are located further into the cavity formed by the shaver housing 203 than the channels 222 containing the pins that grip the shaver. The magnets 228, 230 have positions adjacent to the soft magnetic material 112 of the coupling portion 106 of the shaver.

Further into the cavity than the location of the magnets 228 and 230, a pin 232 protrudes upwards from the bottom of the cavity. The pin 232 engages with a slot 114 in the coupling portion 106 of the shaver. The coupling portion 106 of the shaver also has a slot 117 that is positioned opposite the slot 114 engaged by the pin 232. By engaging one of the slots, the pin 232 fixes the rotational position of the coupling portion 106 and thus the outer tubular portion 104. The shaver can thus be inserted into the handpiece in one of two possible rotational orientations that are 180 degrees apart.

A drive shaft 234 protrudes from the rear of the cavity along the central axis of the cavity. The protruding end of the drive shaft 234 has flats to engage the slits 116 at the end of the inner tubular section 102 of the shaver. A seal 235 encircles the drive shaft 234 to prevent irrigation fluid and debris from entering the motor.

A button assembly 211 is located in a recess in the outer body 205 above the shaver housing 203 and behind the blade release sleeve 202. Two buttons 204, 204 rest against two membrane switches 215, 217. A screw 213 secures button assembly 211 to outer body 205.

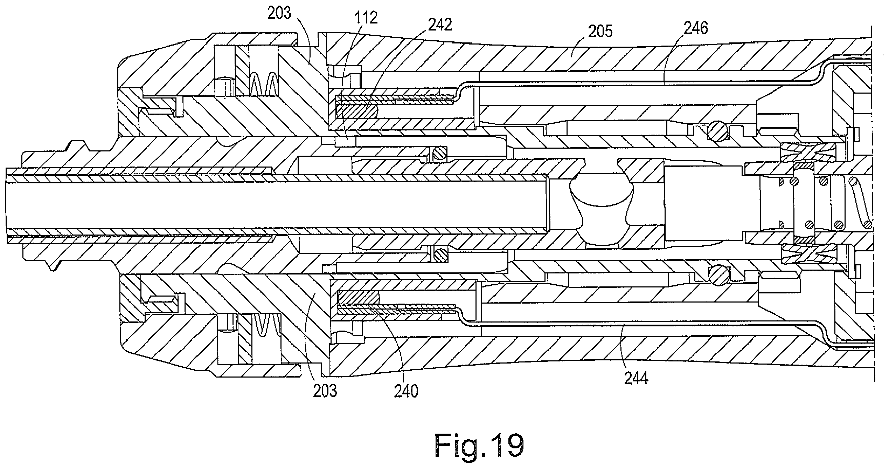

FIG. 19 shows a top sectional view of the front of the handpiece with a shaver attached. Within the shaver housing 203, two potted Hall sensors 240, 242 are located at opposite sides of the cavity formed by the shaver housing 203. The potted Hall sensors 240 and 242 are adjacent to the soft magnetic material 112 on the shaver at opposing sides of the cavity formed by the shaver housing 203. The potted Hall sensors 240, 242 are connected to circuits 244, 246 that run from the Hall sensors 240, 242 towards the rear of the handpiece. The circuits 244 and 246 connect the Hall sensors 240 and 242 respectively to a cable running from the rear of the handpiece.

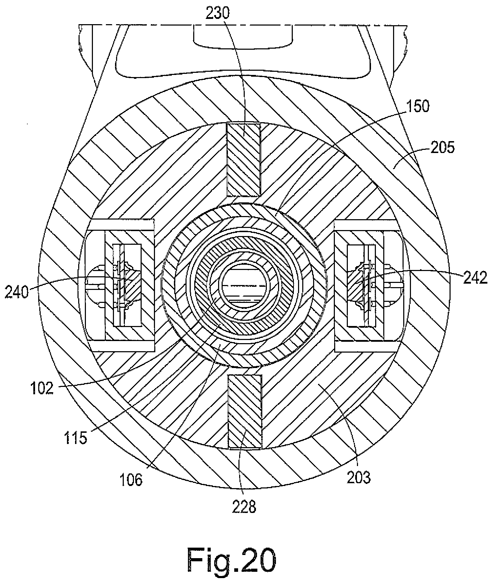

FIG. 20 shows a vertical section of the handpiece showing the locations of the magnets 228, 230, the potted Hall sensors 240, 242 and the soft magnetic material 150. The cross section includes a number of concentric circular elements. The outermost element is the outer body 205 of the handpiece. The Hall sensors 240, 242 are located at opposing horizontal sides of the shaver housing 203... The shaver housing 203 is substantially circular in cross section with the exterior side having a flattened section on each side. The shaver housing 203 has blind holes at the top and bottom, which are cut from the exterior of the shaver housing 203 to receive the magnets 228, 230. The interior face of the shaver housing 203 is circular, whereas the exterior face of the shaver housing has sections cut away to accommodate the potted Hall sensors 240, 242, and the magnets 228, 230.

The shaver housing 203 of the handpiece fixes and protects the magnets 228, 230 and the potted Hall sensors 240, 242 from contact with the shaver blade when the shaver blade is inserted into and removed from the handpiece.

Within the shaver housing 203 is the shaver blade. The outermost layer of the shaver blade 100 is the ring of soft magnetic material 150. Within the soft magnetic material 150, the coupling portion 106 of the outer tubular portion forms a concentric circle in cross section, and is thicker than the soft magnetic material 150.

Inside the coupling portion 106 of the outer tubular portion, the coupling portion 115 of the inner tubular portion and the inner tubular portion 102 form two further concentric circles.

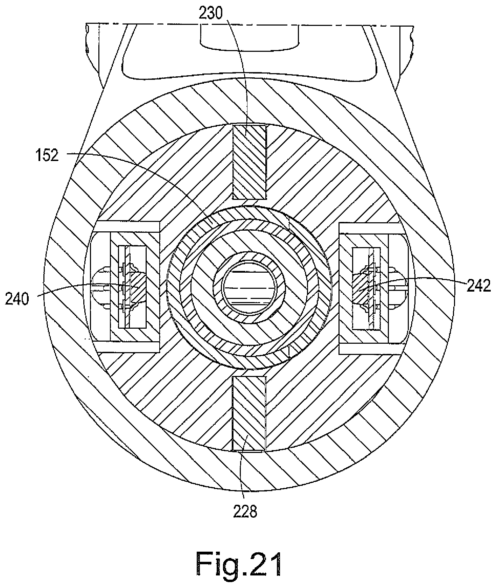

FIG.21 shows a vertical section of the handpiece showing the locations of the magnets 228, 230, the Hall sensors 240, 242 and the soft magnetic material 152. The soft magnetic material 152 has a lesser circumferential extent than the soft magnetic material 150 shown in FIG. 15. The soft magnetic material 152 transverses a section of the circumference of the coupling portion 106 of the outer portion of the shaver 100 through an angle of approximately 240 degrees. In cross section, the soft magnetic material 152 forms a 'C' shape, with an opening of approximately 110 degrees adjacent to one of the Hall sensors 242.

The soft magnetic material 152, thus occupies a circumferential position adjacent to both of the magnets and one of the Hall sensors 240, but does not lie in a circumferential position adjacent to the second Hall sensor 242.

By measuring the magnetic field using the Hall sensors, the handpiece can determine the shape of the soft magnetic material on the shaver blade and thus determine a property of the shaver blade.

## Claims

1. An arthroscopic shaver blade (10; 100) adapted to be mounted on a driving means (20; 200), said arthroscopic shaver blade comprising an outer portion (104) and an inner portion (102) configured to rotate within the outer portion (104) when driven by the driving means (20; 200), the outer portion (104) comprising a coupling portion (11,106) **characterized by** the coupling portion comprising an indicator means (12; 112) for allowing said driving means to determine a property of said arthroscopic shaver blade (10; 100), wherein:
said indicator means comprises a soft magnetic material configured to affect a magnetic field detected in said driving means (20; 200), and wherein the shape and/or extent of the soft magnetic material indicates a property of the arthroscopic shaver blade (10; 100);
said soft magnetic material being arranged substantially in a plane, said plane being substantially perpendicular to the longitudinal axis of said arthroscopic shaver blade;
said soft magnetic material being a ring section located around the longitudinal axis of the arthroscopic shaver blade wherein an angular extent of said soft magnetic material around said longitudinal axis indicates said property of said arthroscopic shaver blade (10; 100); and
said soft magnetic material forms a part of the outer surface of the coupling portion(11, 106) of the outer portion of said arthroscopic shaver blade (10; 100).

2. The arthroscopic shaver blade of claim 1, the soft magnetic material being non-structural.

3. The arthroscopic shaver blade of claim 1, said ring section having an opening, said opening subtending an angle of at least 40 degrees at said longitudinal axis.

4. The arthroscopic shaver blade of any one of the preceding claims, the coupling portion further comprising an orientating means (114) for fixing an orientation of said coupling portion relative to said driving means.

5. The arthroscopic shaver blade of any preceding claim wherein the inner portion comprises a cutting part comprising a burr.

6. A plurality of interchangeable arthroscopic shaver blades, each arthroscopic shaver blade of the plurality of arthroscopic shaver blades being an arthroscopic shaver blade according to claims 1 to 5 configured to be interchangeably mounted on a common driving means, wherein each arthroscopic shaver blade has a difference in a property and each indicator means is configured to differentiate between arthroscopic shaver blades to indicate said difference in said property.

## Patentansprüche

1. Arthroskopische Schabklinge (10; 100) angepasst, um auf einem Antriebsmittel (20; 200) montiert zu werden, wobei die arthroskopische Schabklinge einen äußeren Teil (104) und einen inneren Teil (102) umfasst, konfiguriert zum Rotieren innerhalb des äußeren Teils (104), wenn er durch das Antriebsmittel (20; 200) angetrieben wird, wobei der äußere Teil (104) einen Kupplungsteil (11; 106) umfasst, **dadurch gekennzeichnet, dass** der Kupplungsteil ein Anzeigemittel (12; 112) umfasst, das es dem Antriebsmittel ermöglicht eine Eigenschaft der arthroskopischen Schabklinge (10; 100) zu ermitteln, wobei:
das Anzeigemittel ein weichmagnetisches Material umfasst, konfiguriert, um ein in dem Antriebsmittel (20; 200) detektiertes Magnetfeld zu beeinflussen; und wobei
die Form und/oder Ausdehnung des weichmagnetischen Materials eine Eigenschaft der arthroskopischen Schabklinge (10; 100) anzeigt;
wobei das weichmagnetische Material im Wesentlichen in einer Ebene angeordnet ist, wobei die Ebene im Wesentlichen senkrecht zu der Längsachse der arthroskopischen Schabklinge liegt;
wobei das weichmagnetische Material eine Ringsektion ist, die um die Längsachse der arthroskopischen Schabklinge angeordnet ist, wobei eine Winkelausdehnung des weichmagnetischen Materials um die Längsachse die Eigenschaft der arthroskopischen Schabklinge (10; 100) anzeigt; und
wobei das weichmagnetische Material einen Bestandteil der äußeren Oberfläche des Kupplungsteils (11, 106) des äußeren Teils der arthroskopischen Schabklinge (10 ;100) bildet.

2. Arthroskopische Schabklinge nach Anspruch 1, wobei das weichmagnetische Material ein Nicht-Struktur-Material ist.

3. Arthroskopische Schabklinge nach Anspruch 1, wobei die Ringsektion eine Öffnung hat, wobei die Öffnung an der Längsachse einen Winkel von mindestens 40 Grad einschließt.

4. Arthroskopische Schabklinge nach einem der vorhergehenden Ansprüche, wobei das Kupplungsmittel ferner ein Ausrichtungsmittel (114) zum Fixieren einer Ausrichtung des Kupplungsmittels im Verhältnis zu dem Antriebsmittel umfasst.

5. Arthroskopische Schabklinge nach einem der vorhergehenden Ansprüche, wobei der innere Teil einen Schneidteil umfasst, der eine Fräse aufweist.

6. Vielzahl von austauschbaren arthroskopischen Schabklingen, wobei jede arthroskopische Schabklinge der Vielzahl von artrhoskopischen Schabklingen eine arthroskopische Schabklinge nach den Ansprüchen 1 bis 5 ist, konfiguriert, um austauschbar auf einem gebrauchsüblichen Antriebsmittel montiert zu werden, wobei jede arthroskopische Schabklinge einen Unterschied in einer Eigenschaft aufweist und jedes Anzeigemittel konfiguriert ist, um die arthroskopischen Schabklingen voneinander zu unterscheiden, um den Unterschied in der Eigenschaft anzuzeigen.

## Revendications

1. Lame de rasoir arthroscopique (10; 100) adaptée pour être montée sur un moyen d'entraînement (20; 200), ladite lame de rasoir arthroscopique comprenant une partie extérieure (104) et une partie intérieure (102) configurée pour tourner à l'intérieur de la partie extérieure (104) lorsqu'elle est entraînée par le moyen d'entraînement (20; 200), la partie extérieure (104) comprenant une partie d'accouplement (11, 106) **caractérisée en ce que** la partie d'accouplement comprend un moyen indicateur (12; 112) pour permettre audit moyen d'entraînement de déterminer une propriété de ladite lame de rasoir arthroscopique (10; 100), dans laquelle:
ledit moyen indicateur comprend un matériau magnétique doux configuré pour affecter un champ magnétique détecté dans ledit moyen d'entraînement (20; 200), et dans lequel
la forme et/ou l'étendue du matériau magnétique doux indique une propriété de la lame de rasoir arthroscopique (10; 100);
ledit matériau magnétique doux étant disposé sensiblement dans un plan, ledit plan étant sensiblement perpendiculaire à l'axe longitudinal de ladite lame de rasoir arthroscopique;
ledit matériau magnétique doux étant une section d'anneau située autour de l'axe longitudinal de la lame de rasoir arthroscopique dans laquelle une étendue angulaire dudit matériau magnétique doux autour dudit axe longitudinal indique ladite propriété de ladite lame de rasoir arthroscopique (10; 100); et
ledit matériau magnétique doux fait partie de la surface extérieure de la partie de couplage (11, 106) de la partie extérieure de ladite lame de rasoir arthroscopique (10; 100).

2. Lame de rasoir arthroscopique selon la revendication 1, le matériau magnétique doux étant non structurel.

3. Lame de rasoir arthroscopique selon la revendication 1, ladite section annulaire ayant une ouverture, ladite ouverture sous-tendant un angle d'au moins 40 degrés au niveau dudit axe longitudinal.

4. Lame de rasoir arthroscopique selon l'une quelconque des revendications précédentes, la partie d'accouplement comprenant en outre un moyen d'orientation (114) pour fixer une orientation de ladite partie d'accouplement par rapport audit moyen d'entraînement.

5. Lame de rasoir arthroscopique selon l'une quelconque des revendications précédentes, dans laquelle la partie intérieure comprend une partie coupante comprenant une bavure.

6. Pluralité de lames de rasoir arthroscopiques interchangeables, chaque lame de rasoir arthroscopique de la pluralité de lames de rasoir arthroscopiques étant une lame de rasoir arthroscopique selon les revendications 1 à 5 configurée pour être montée de manière interchangeable sur un moyen d'entraînement commun, dans laquelle chaque lame de rasoir arthroscopique a un différence dans une propriété et chaque moyen indicateur est configuré pour différencier les lames de rasoir arthroscopiques pour indiquer ladite différence dans ladite propriété.
